# EUROPÄISCHE PATENTANMELDUNG

(11) **EP 0 898 949 A1**
(43) Veröffentlichungstag der Anmeldung: **03.03.1999**
(21) Anmeldenummer: 98810831.2
(22) Anmeldetag: 24.08.1998
(51) Int. Cl.: A61F 13/10, A61F 13/14, A61F 5/01

(54) **Armbandage und Verfahren zum Anlegen derselben**

(30) Priorität: 27.08.1997 CH 2002/97
(71) Anmelder: Zihlmann, Wilfried, 8046 Zürich (CH)
(72) Erfinder: Zihlmann, Wilfried, 8046 Zürich (CH)
(74) Vertreter: Legland, Brynjulv

(57) **Zusammenfassung**

Die beschriebene Armbandage (1) ist einstückig ausgebildet und durch Klettband und/oder Druckknöpfe am Rücken des Trägers verbunden bzw. befestigt. Die Halterung des Armes erfolgt über einen Armträger (4), wobei die Halterung durch einen Seitenträger (5) und einen Schrägträger (3) unterstützt wird.

Die Armbandage (1) ist unkompliziert in der Ausführung, in einem Arbeitsgang herstellbar und lässt sich schnell und einfach am Körper eines Patienten ohne medizinische Spezialkenntnisse anbringen.

Der entscheidende Vorteil gegenüber herkömmlichen Ausführungen liegt darin, dass sie keine unangenehmen Nachwirkungen auf den Körper des Patienten - auch nach längerem Tragen - verursacht.

## Beschreibung

Die Erfindung betrifft eine Armbandage gemäss dem Oberbegriff des ersten Patentanspruches, sowie ein Verfahren zu deren Herstellung.

Derartige Bandagen werden zur Halterung und Fixation eines Armes oder einer Schulter, der bzw. die durch Verletzungen oder Brüche, insbesondere im Schulter-, proximalen Oberarm- und Ellbogen-Bereich, festgehalten werden muss, damit die Arm- oder Schulter-Verletzung schneller heilt oder zusammenwächst. Ferner werden solche Bandagen dazu benutzt, einen Arm ruhig zu halten, wenn er nur verstaucht ist und keine Brüche aufweist.

In der ursprünglichen Form bestanden solche Armbandagen oder Armschlingen z.B. aus einem Halstuch, das an den beiden Enden zusammengebunden ist. Verbesserte Ausführungen solcher Armschlingen sind unten mit einem Tuch versehen, das etwa die Länge des Unterarmes aufweist.

Der meistens mittels einer Aluminiumschiene oder eines aufblasbaren Kunststoffkörpers abgestützte und ausgerichtete, verletzte Arm wird in solchen Armbandagen getragen.

Bekannte Armbandagen sind mit dem Nachteil behaftet, dass sie am Nacken des Trägers abgestützt sind. Da eine länger andauernde Abstützung am Nacken des Trägers sehr häufig mit Nackenschmerzen verbunden ist, die eine physiotherapeutische Nachbehandlung erfordern, wird eine Befestigungsart gesucht, welche diesen Umstand beseitigt.

Ferner berücksichtigen bestehende Ausführungen nicht den weiblichen Körperbau ausreichend, so dass der Tragriemen einen Druck gegen die Brüste ausübt.

Für eine stillende Frau ist es fast unmöglich dies zu tun, wenn sie die bisher üblichen Armbandagen benutzt.

Da die Heilung einer Armverletzung, z.B. nach einer Operation des Schulter- oder Armgelenkes, drei Wochen oder mehr beanspruchen kann, ist es wichtig, dass ein Patient, Mann oder Frau mit einer formgerechten und bequemen Armbandage versehen ist, nach deren Entfernung keine medizinische Nachbehandlung der durch das Tragen verursachten Schäden benötigt wird.

Aufgabe der Erfindung ist somit die Schaffung einer Armbandage, die sowohl den Körperbau von Frauen als auch von Männern berücksichtigt und nicht die Nachteile der bestehenden Ausführungen aufweist.

Ferner ist es Aufgabe der Erfindung, ein Verfahren zur Herstellung der Armbandage zu schaffen.

Dabei soll der Nacken, insbesondere auf der dem unterstützten Arm gegenüberliegenden Seite, nicht belastet und somit eine Versteifung oder Verkrampfung dieser Nackenseite verhindert werden.

Ferner soll die Armbandage den Arm gegen den Körper festhalten, damit er nicht in Schwingung kommt, wenn sich der Träger nach vorne oder hin- und herbewegt.

Diese Aufgaben sind erfindungsgemäss durch die Merkmale im Kennzeichnungsteil des ersten und des zweiten Patentanspruches gelöst.

Ausführungsformen sind in den abhängigen Ansprüchen umschrieben.

Nachfolgend wird ein Ausführungsbeispiel der erfindungsgemässen Armbandage anhand der Zeichnung näher erläutert. Es zeigen:
- Fig. 1: eine schematische Draufsicht einer Armbinde vor dem Anlegen an einen Patienten,
- Fig. 2: eine Vorderansicht des rechts an einem Patienten angelegten Armbind in einer ersten Stufe des Anlegens,
- Fig. 3: wie Fig. 2, jedoch von hinten, mit loser Seitenhalterung,
- Fig. 4: wie Fig. 2, jedoch in einer zweiten Stufe, nach dem Anlegen am rechten Arm,
- Fig. 5: wie Fig. 4, jedoch von hinten,
- Fig. 6: wie Fig. 4, jedoch in einer dritten Stufe, von vorne,
- Fig. 7: wie Fig. 6, jedoch von hinten, und
- Fig. 8: die fertig angelegte Armbinde von hinten.

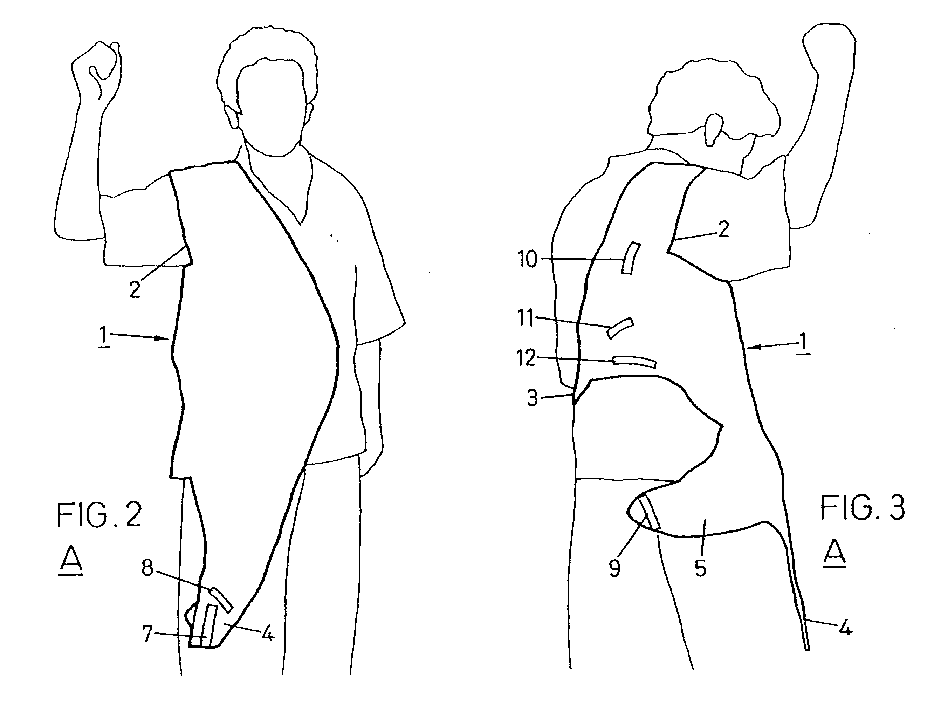

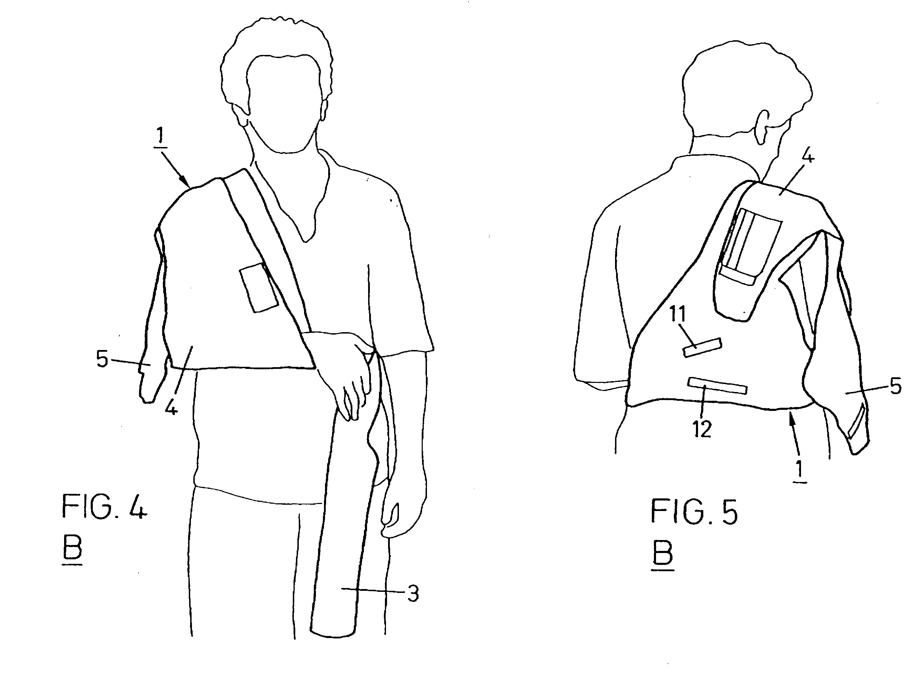

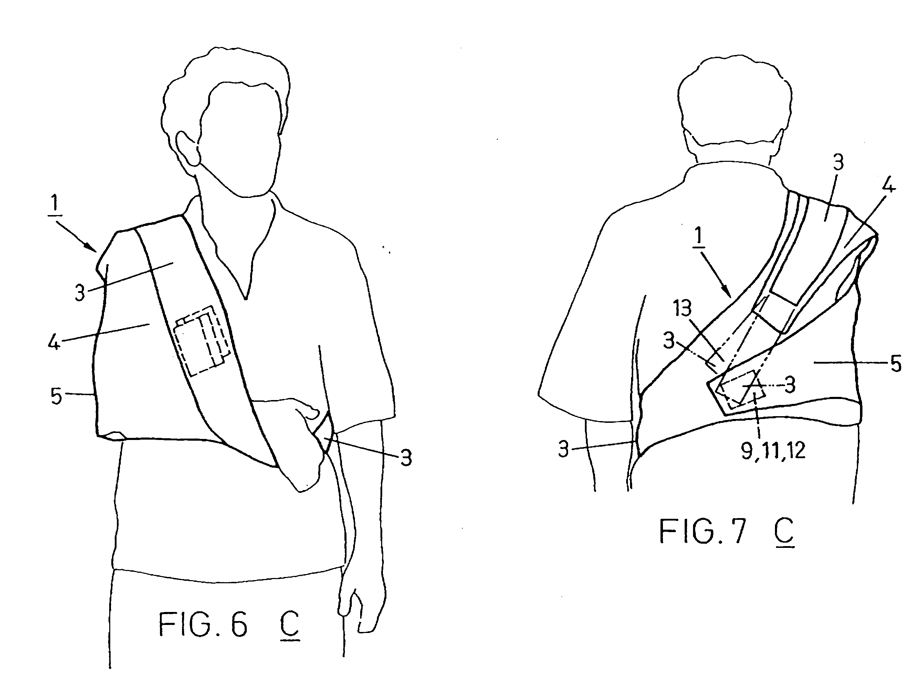

In den Figuren bezeichnen die einzelnen Stufen A bis D die verschiedenen Schritte zum Anlegen der Armbinde, wobei A in den Fig. 2 und 3, B in Fig. 4 und 5, C in den Fig. 6 und 7, und D in Fig. 8 dargestellt sind.

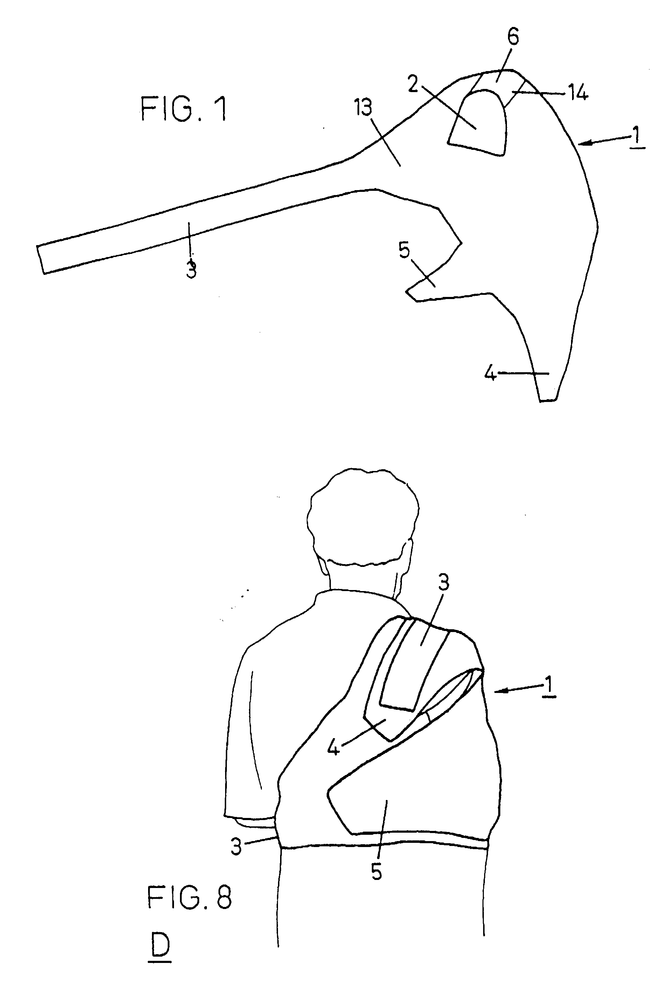

In Fig. 1 ist eine schematische Draufsicht einer Armbinde 1 mit einem Armloch 2, einem Schrägträger 3, einem Armträger 4, und einer horizontal anzuordnenden Seitenhalterung gezeigt, wobei die Stelle 6 auf die Achsel eines Patienten zu liegen kommt.

Fig. 2 zeigt die rechts am Patienten angeordnete Armbinde 1, wobei der mit Klettbändern 7,8 ausgestattete Armträger 4 nach vorne hinunterhängt, während die horizontale Seitenhalterung 5 und der noch nicht befestigte Schrägträger 3 hinter dem Rücken des Patienten zu liegen kommen und somit unsichtbar sind.

Fig. 3 zeigt den Rücken des Patienten und somit die Seitenhalterung 5 mit einem Klettband 9 sowie einen kleinen Teil des Schrägträgers 3, der in dieser Fig. nach vorne gezogen ist. Im Rückenbereich 13 (Fig. 1) befinden sich drei Klettbänder 10-12 zur Befestigung des Armträgers 4 und der Seitenhalterung 5.

In Fig. 4 ist der Arm mittels des Armträgers 4 in abgewinkelter Lage gehalten, der am Rücken des Patienten mittels eines Klettbandes 9 befestigt ist, das in dasjenige 10 eingreift (Fig. 5).

Fig. 6 zeigt eine Vorderansicht des Patienten, in welcher die Seitenhalterung 5 mittels der Klettbänder 9, 11 und 12 am Rücken des Patienten befestigt ist (Fig. 7). Dann wird der Schrägträger 3 angelegt, der über den Armträger 4 über die Schulter zum Rücken zieht und dort befestigt ist, und zwar entweder nur auf dem Ende des Armträgers 4 oder noch zusätzlich auf der Seitenhalterung 5, oder einfach nur im Rückenbereich 13 der Armbinde.

Mit Bezug auf Fig. 7 und 8 wird darauf hingewiesen, dass der Schräggürtel 3 normalerweise am Schluss des Anbringens der Armbinde montiert wird. Es ist aber in einer abweichenden Ausführung möglich, zuerst den Schrägträger 3 und nachher die Seitenhalterung 5 am Rücken des Patienten zu befestigen.

Bei der beschriebenen Armbandage kann der Patient einen im Gips fixierten Arm tragen, wobei genügend Raum zum Einsetzen einer Fusion vorhanden ist. Der Ellbogen kann auch in einer gepolsterten Schale liegen, die in der Bandage integriert ist.

Für die Herstellung der einzelnen Verbindungen wird z.B. Klettband benutzt, so dass das Anbringen der Bandage leicht und schnell durchführbar ist. Es ist aber möglich, Druckknöpfe, evt. in Verbindung mit Klettband zu verwenden.

Für Frauen ist es wichtig, dass der Schrägträger 3 zwischen den Brüsten zu liegen kommt.

Die beschriebene Ausführung ist für die Herstellung in den üblichen Textilgrössen S, M, L und XL vorgesehen. Da Klettbandvorrichtungen zwischen den einzelnen Bändern vorgesehen sind, ist es bei einer bestimmten Grösse ohne Mehrarbeit möglich, Anpassungen an die Masse der Trägerin oder des Trägers vorzunehmen.

Jede Grösse (S, M, L und XL) ist sowohl für den rechten als auch für den linken Arm verwendbar.

Das Anlegen der Bandage kann vom Träger allein durchgeführt werden, sofern dies aus medizinischen Gründen möglich bzw. ratsam ist.

Die Armbandage ist z.B. für Distorsion, Kontusion, Rotatorenmanschetten-Verletzungen, Periarthritis, Humeroscapularis, Luxation und Luxationsfrakturen im Schulterbereich vorgesehen.

Für den Oberarm ist die Bandage ferner für subcapitale Humerusfrakturen nach operativen Eingriffen geeignet, die nicht unbedingt operiert werden müssen.

Die beschriebene Bandage für postoperative und posttraumatische Einsätze hat u.a. die folgenden Vorteile:
- sichere und stabile Fixation der Schulter und des Oberarmes
- mittels Klettbandverschlusses einfach und schnell anlegbar
- beidseits (links und rechts) tragbar
- engt nicht am Thorax ein
- erleichtert die Krankengymnastik und Körperpflege
- eine Schulter mit Nackenpartie ist immer frei

Sollte sich der Patient oder die Patientin, beispielsweise mit der Bandage unwohl fühlen, kann er oder sie diese ohne Hilfe lösen oder verschieben.

Der vielleicht wichtigste Vorteil der vorliegenden Armbandage gegenüber bestehenden Ausführungen liegt darin, dass die Nackenpartie geschont wird. Bisher wurden häufig, insbesondere Frauen, nach relativ kurzem Tragen einer Armbinde von Nackenschmerzen geplagt, so dass eine physiotherapeutische Behandlung notwendig wurde. Diese Behandlung war, abgesehen von den Schmerzen, für die betreffende Person mit einer grossen finanziellen Belastung verbunden.

Mit der beschriebenen Armbinde wird der Körperbau der Frauen berücksichtigt, so dass sie für eine stillende Frau keine Probleme bereitet.

Die Armbinde wird vorzugsweise aus einem Textiltuch ausgeschnitten, wobei eine Anpassung an den menschlichen Körper im Achselbereich zweckmässig ist, was z.B. darin bestehen kann, dass dieser Bereich aufgeschnitten und mit einer Polsterung versehen wird. Das Material für die Armbandage kann z.B. aus Baumwolle und/oder Kunststoff bestehen.

Zum schnelleren Anlegen, auch für ungeübte, ist es vorgesehen, die einzelnen Teile, insbesondere 3 bis 5 und 7 bis 11 mit unterschiedlichen Farben zu versehen. Ferner ist es vorgesehen, für einander bestimmte Klettbänder mit gleichen Farben oder Nummern zu versehen.

## Patentansprüche

1. Einstückige Armbandage zur Halterung und Fixation eines abgewinkelten Armes oder einer Schulter am Körper eines Patienten, dadurch gekennzeichnet, dass sie ein Armloch (2) mit einer Achselauflage (6), einem Armträger (4), eine Seitenhalterung (5) sowie einen Schrägträger (3) aufweist, die jeweils durch Vorrichtungen (7-12) lösbar miteinander verbunden sind.

2. Verfahren zur Herstellung der Armbinde nach Anspruch 1, dadurch gekennzeichnet, dass man zuerst den Arm des Patienten durch das Armloch führt, und den Arm abgewinkelt mittels des Armträgers an den Körper anlegt, wobei der Armträger rückseitig am Patienten befestigt wird, dass ferner die horizontale Seitenhalterung nach hinten bewegt und am Rücken befestigt wird, und dass schliesslich der Arm mittels des Schrägträgers am Körper angelegt wird.

3. Armbandage nach Anspruch 1, dadurch gekennzeichnet, dass sie Baumwolle und/oder Kunststoff aufweist.

4. Armbandage nach Anspruch 1 oder 3, dadurch gekennzeichnet, dass sie im Achselbereich (6) eine Polsterung (14) aufweist.

5. Armbandage nach Anspruch 1, 3 oder 4 dadurch gekennzeichnet, dass sie im Achselbereich (6) eine dem Schulterverlauf angepasste Abwinkelung aufweist

6. Armbandage nach Anspruch 1, 3, 4 oder 5, dadurch gekennzeichnet, dass die Vorrichtungen (7-12) jeweils ein Klettband und/oder ein Druckknopf aufweisen.

7. Armbandage nach einem der Ansprüche 1 oder 3-6, dadurch gekennzeichnet, dass die verschiedenen Teile 3-5 und 7-12, zur Erleichterung des Anornens an einem Patienten, unterschiedliche Farben aufweisen.

8. Armbandage nach einem der Ansprüche 1 oder 3-7, dadurch gekennzeichnet, dass die ineinander eingreifenden Klebstreifen die gleiche Farbe oder die gleiche Nummer aufweisen.

9. Verfahren nach Anspruch 2, dadurch gekennzeichnet, dass man die einzelnen Teile am Rücken des Patienten mittels Klettband und/oder Druckknöpfe befestigt.
